# EUROPÄISCHE PATENTSCHRIFT

(11) **EP 1 013 079 B1**
(45) Veröffentlichungstag und Bekanntmachung des Hinweises auf die Patenterteilung: **13.06.2001**
(21) Anmeldenummer: 98946311.2
(22) Anmeldetag: 10.08.1998
(51) Int. Cl.: H04N 5/217, A61B 6/03, A61B 6/14

(54) **VERFAHREN ZUR KOMPENSATION DES DUNKELSTROMS EINES MEHRERE PIXEL AUFWEISENDEN ELEKTRONISCHEN SENSORS**
METHOD FOR COMPENSATING THE DARK CURRENT OF AN ELECTRONIC SENSOR HAVING SEVERAL PIXELS
PROCEDE POUR LA COMPENSATION DU COURANT D'OBSCURITE D'UN DETECTEUR ELECTRONIQUE REPRESENTANT PLUSIEURS PIXELS

(30) Priorität: 11.08.1997 DE 19734717
(43) Veröffentlichungstag der Anmeldung: 28.06.2000
(73) Patentinhaber: Sirona Dental Systems GmbH, 64625 Bensheim (DE)
(72) Erfinder: BONK, Roland, D-76297 Stutensee (DE); ZELLER, Uwe, D-61267 Anspach (DE)
(74) Vertreter: Sommer, Peter
(86) Internationale Anmeldenummer: EP9805069
(87) Internationale Veröffentlichungsnummer: WO9908440

(56) Entgegenhaltungen:
- WO-A-89/10037
- WO-A-93/23952
- DE-A- 19 604 631

## Beschreibung

Die Erfindung betrifft ein Verfahren zur Kompensation des Dunkelstroms eines mehrere Pixel aufweisenden elektronischen Sensors. Dabei ist aus einer Strahlenquelle eine Strahlung zur Erzeugung eines Bildsignals auf einen den Sensor enthaltende Detektoranordnung gerichtet und es werden mehrere Aufnahmen mit unterschiedlicher Aufnahmezeit (Integrationszeit) durchaus Auslesen des in den Pixeln des Sensors vorhandenen Detektorsignals erstellt. Ein derartiges Verfahren ist insbesondere zur Erstellung von zahnärztlichen Panorama- oder cephalometrischen Schichtaufnahmen mit einem Röntgendiagnostikgerät, welches eine Dreheinheit mit einer Strahlenquelle und einer diametral dazu angeordneten Detektoranordnung mit elektronischem Sensor, vorzugsweise einem CCD-Sensor, enthält, verwendbar.

Aus der EP-A-0 279 294 ist es bekannt, in der zahnärztlichen Röntgendiagnostiktechnik bei Panorama- oder cephalometrischen Röntgenschichtaufnahmen anstelle eines Films mit Verstärkerfolie einen strahlenempfindlichen (CCD-)Sensor einzusetzen und durch eine besondere Betriebsart des Sensors (TDI: Time Delay and Integration) die Funktion des bewegten Filmes elektronisch "nachzubilden", indem die durch die Belichtung erzeugten Ladungspakete des Sensors entsprechend weitergetaktet werden, während ständig neue Ladungen hinzukommen.

Auch die DE-A-19 525 678 beschreibt eine Methode und eine Vorrichtung zur Einstellung von bilderzeugenden Werten in einem Panorama-Röntgenbild erzeugenden Apparat. Nachteilig ist hier, daß die durch den Herstellprozeß bedingte Ortsabhängigkeit des Dunkelstroms in Bezug auf die einzelnen Pixel nicht berücksichtigt. Zwar ist durch die Regelung des Signal-Rausch-Abstandes implizit eine Zeitabhängigkeit gegeben, die beschriebene Anordnung setzt aber einen idealen Sensor voraus, der ohne Fehlstellen in den Pixeln arbeitet.

Der Röntgensensor besteht konstruktiv in der Regel aus mehreren, mit minimalstem Abstand angeordneten (CCD-)Elementen. Dem durch Röntgenstrahlung erzeugten Bildsignal sind verschiedene Störsignale überlagert. Die dominierende Beeinträchtigung des Signals erfolgt durch das zeit- und temperaturabhängige Generieren von Ladungsträgern im Sensor, das in der einschlägigen Literatur "Solid State Imaging with Charge-Compled Devices", Albert J.P. Theuwissen, Kluwer Academic Publishes Dordrecht, Boston, London 1995, Seite 92-95 (und Seiten 274/275 zum TDI-Betrieb) als Dunkelstrom bezeichnet wird. Das generierte Dunkelstromsignal ist sowohl vom eingesetzten Sensor als auch von den einzelnen Pixeln des Sensors abhängig; das Dunkelstromsignal ist damit ortsabhängig [y]. Weiterhin ist dieses Signal von der Integrationszeit und dadurch auch von der variablen Umlaufgeschwindigkeit des Drehsystems abhängig und somit zeitabhängig [x]. Im übrigen ist das Dunkelstromsignal auch abhängig von der Temperatur und der über die Lebensdauer kummulierten Röntgendosis.

Bisher wird man eine Korrektur des Dunkelstroms dadurch erzielen, indem man zu Beginn und Ende einer Aufnahme eine Dunkelstrominformation aus den abgedeckten bzw, unbestrahlten Randzonen des Sensors erfaßt. Aus den so gewonnenen Daten wird für jede Zeile einen Korrekturwert berechnet. Von jedem Pixel einer Zeile subtrahiert man anschließend den dazugehörigen, über die gesamte Zeile konstanten Korrekturwert.

Nachteil dieser Dunkelstromkorrektur ist, daß zwar Schwankungen der Integrationszeit, und damit die zeitabhängige Änderung des Dunkelstromes, berücksichtigt werden, aber keine Schwankungen der verschiedenen Pixel einer Zeile des Sensors. Es können somit in Abhängigkeit vom verwendeten CCD-Sensor signifikante Bildartefakte auftreten.

Eine weitere bekannte Methode zur Dunkelstromkorrektur ist die Subtraktion einer kompletten Dunkelstromaufnahme. Dazu wird zusätzlich zur Bildaufnahme eine Dunkelstromaufnahme durchgeführt und von der Strahlungsaufnahme subtrahiert. Obgleich eine solche Methode gute Ergebnisse liefert, ist sie für die eingangs erwähnten Panorama- und cephalometrischen Aufnahmen nicht anwendbar, da durch das Dunkelstrombild und des damit verbundenen zusätzlich erforderlichen Speicheraufwandes sich die Kosten in unangemessener Weise erhöhen würden. Außerdem würde sich der Aufnahmeablauf durch die erforderliche Wartezeit, die durch die Erzeugung der Positionsimpulse notwendig ist, verzögern.

Aus der WO-A-8 910 037 ist ein Verfahren und eine Vorrichtung zur Kompensation des Dunkelstroms und einer Grundwertverschiebung der Spannung einer CCD-Einheit bekannt, bei dem eine Regelstrecke eingesetzt wird, deren Stellgröße von verdeckt angeordneten CCD-Zellen abgeleitet wird. Dieser Vorgang wird ständig für jede Zeile des linearen CCD wiederholt und die Dunkelstrominformation der verdeckten CCD-Zellen wird zur Korrektur physikalisch an anderer Stelle angeordneter CCD-Zellen verwendet. Dabei wird die Integrationseinheit mit zwei Zeitkonstanten betrieben. Dazu werden die Signale von verdeckt angeordneten CCD-Zellen verstärkt und in digitale Signale umgewandelt. In einer Vergleichseinheit werden die digitalen Werte mit einem vorgegebenen Wert verglichen. Ist der aus den verdeckten Zellen stammende Wert zu hoch, so wird die Zeitkonstante
der Integrationseinheit verringert. Nähert sich der digitalisierte Wert dem Optimum, so wird die Integrationseinheit mit einer großen Zeitkonstante betrieben, was ein schnelleres Auslesen zur Folge hat. Weiterhin wird vorgeschlagen, den Integrationseinheiten eine so hohe Zeitkonstante zuzuordnen, daß das verstärkte Ausgangssignal während des Auslesens einer Zeile aus der CCD-Einheit in etwa konstant bleibt. Gleichwohl ist ein Umschalten der Zeitkonstante der Integrationseinheit von einer ersten in eine zweite Position mit kleiner Zeitkonstante möglich, um die Kontrollschleife auszuführen, wenn man von der optimalen Dunkelstromkompensation weit entfernt ist, wie dies beispielsweise beim Aufwärmen des Gerätes oder bei einer starken Schwankung der Umgebungstemperaturen der Fall ist.

Nachteilig ist auch hier, daß nur die verdeckt angeordneten CCD-Zellen für einen Hinweis auf das Dunkelstrom-Profil und die erforderliche Dunkelstromkompensation herangezogen werden, nicht jedoch die eigentlichen für die Bildaufnahme verwendeten CCD-Zellen selbst. Weiterhin ergibt sich durch die notwendige Zeit zum Vergleich der digitalen Werte mit einem vorgegebenen Wert zu Beginn jeder Bildzeile eine Wartezeit, in der keine Bilddaten erfaßt werden.

Ziel der vorliegenden Erfindung ist es, demgegenüber eine verbesserte Lösung einer Kompensation des Dunkelstromes zu erzielen.

Erfindungsgemäß wird vor Beginn oder nach dem Ende einer Aufnahme der Sensor ohne Strahlung mit wenigstens zwei unterschiedlichen Austaktgeschwindigkeiten (Integrationszeiten) ausgelesen und damit für jedes Pixel wenigstens zwei Dunkelstromsignale erfaßt werden. Anschließend werden die ausgelesenen Dunkelstromsignale zur Berechnung eines Dunkelstromwertes der einzelnen Pixel in Abhängigkeit der Austaktgeschwindigkeit herangezogen und damit ein für jeden Bildpunkt (Pixel) verfügbare berechneter Dunkelstromwert bestimmt. Dieser Dunkelstromwert wird schließlich zur Bildkorrektur verwendet, indem anschließend mit dem jedem Pixel zugeordneten Dunkelstromwert eine Korrektur des mit dem Dunkelstromsignal überlagerten Detektorsignals zur Ermittlung des Bildwerts vorgenommen wird.

Vorteilhafterweise wird ein CCD-Sensor mit diesem Verfahren betrieben, wobei insbesondere der Betrieb einer zweidimensionalen Pixelmatrix im TDI-Modus von besonderer Bedeutung ist.

Der Sensor kann dabei mehrere voneinander räumlich abgesetzte Bereiche aufweisen, zwischen denen nicht unbedingt eine Signalerfassung stattfindet. Vorteilhafterweise ist dieser nicht strahlungsempfindliche Bereich minimal, etwa in der Größenordnung eines Pixels (Bildpunkts). Dadurch läßt sich ein aus mehreren Bereichen zusammengesetzter Sensor herstellen. Das insbesondere im Randbereich jedes Bereichs gegenüber den mittigen Bereichen abweichende Verhalten kann durch das erfindungsgemäße Verfahren korrigiert werden.

Für die Berechnung der Abhängigkeit des Dunkelstromwertes von der Austaktgeschwindigkeit wird anhand der mindestens zwei ausgelesenen Dunkelstromsignale eine rechnerische Beziehung hergestellt und der einen Pixel zugeordnete Dunkelstromwert mittels einer Inter- oder Extrapolation entsprechende rechnerischen Beziehung in Abhängigkeit von der tatsächlichen Aufnahmezeit berechnet. Die Anzahl der ausgelesenen Doppelstromsignale für jedes Pixel, kann im mathematischen Sinne als Anzahl von Stützstellen zur Ermittlung einer rechnerischen Beziehung angesehen werden. Das Dunkelstromverhalten eines Pixels wird dabei durch eine Näherung definiert und der Dunkelstromwert eines Pixels in Abhängigkeit von der Integrationszeit als Gleichung erster Ordnung, die durch zwei Stützstellen definiert ist, bzw. als Ausgleichsfunktion höherer Ordnung, sofern mehr als zwei Stützstellen erfaßt werden, ausgedrückt.

Zur Ermittlung der Integrationszeiten der einzelnen Bildspalten werden die Zeiten der Austaktimpulse und damit die Austaktfrequenz erfaßt und festgehalten. Die Integrationszeit einer Bildspalte im TDI-Betrieb ist die Summe der jeweils letzten (n) ermittelten Austaktzeiten, wobei (n) hierbei die Anzahl der Pixel in TDI-Richtung definiert. Die Anzahl der Austaktzeiten ist abhängig von der Breite des Sensors und damit von der Anzahl der Pixel in einer Spalte. Bei einem fiktivem Sensor von beispielsweise 66 Pixeln Breite (in TDI-Richtung) ergeben sich 66 Austaktimpulse. Die Integrationszeiten werden für jede Bildzeile aus vorhergehenden 66 Austaktzeilen berechnet und gespeichert.

Das Verfahren eignet sich insbesondere zur Erstellung einer zahnärztlichen Röntigenaufnahme, da hier eine nichtkonstante Bewegungsgeschwindigkeit des Sensors zu unterschiedlichen Austaktgeschwindigkeiten (Integrationszeiten) des Sensors führt.

Vorteilhafterweise wird mit dem erfindungsgemäßen Verfahren eine Erstellung von zahnärztlichen Panorama- oder cephalometrischen Schichtaufnahmen mit einem Röntgendiagnostikgerät vorgenommen, welches eine Dreheinheit mit einer Strahlenquelle und einer diametral dazu angeordneten Detektoranordnung mit mindestens einem elektronischen Sensor enthält. Bei diesen Aufnahmen wird die Dreheinheit mit einer nichtkonstanten Bewegungsgeschwindigkeit um den Kopf des Patienten herumgeführt.

Vorteilhafterweise werden die beim Auslesen des Sensor entstehenden Detektorsignale in einer Speichereinheit abgespeichert. In einem nachfolgenden Schritt wird Korrektur des mit dem Dunkelstromsignal überlagerten Detektorsignals zur Ermittlung des Bildsignals in einer mit der Speichereinheit verbundenen Recheneinheit vorgenommen. Hierdurch wird die Erstellung der korrigierten Aufnahme nach der Erzeugung der unkorrigierten Aufnahme von dem eigentlichen Aufnahmevorgang abgekoppelt, so daß die Aufnahme selbst durch die Korrektur nicht beinflußt wird.

Prinzipiell ist es aber möglich, die Dunkelstromsignale vor Beginn der Aufnahme zu erfassen und eine Echtzeitkorrektur vorzunehmen, so daß bereits während der Aufnahme Bildwerte angezeigt werden können.

Anhand der Fig. 1 bis 5 wird der Ablauf näher erläutert.

Es zeigt die
- Fig. 1: eine Prinzipdarstellung eines zahnärztlichen Röntgendiagnostikgerätes zur Erstellung von Panoramaschichtaufnahmen, die
- Fig. 2: eine Detektoranordnung mit einem mehrere Bereiche aufweisenden Sensor, die
- Fig. 3: ein Dunkelstromprofil aus den Dunkelstromwerten einer Reihe von Pixeln der Detektoranordnung aus Fig. 2, die
- Fig. 4: ein erstes Ablaufschema mit dem grundsätzlichen Prinzip der Erfindung und die
- Fig. 5: ein Ablaufschema unter Verwendung eines Speichers.

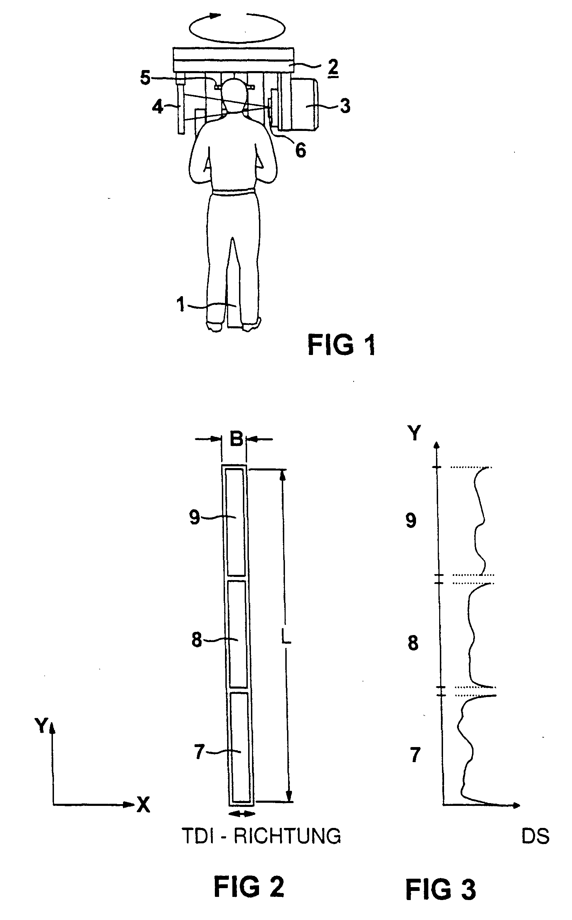

Die Fig. 1 zeigt in einer Prinzipdarstellung ein zahnärztliches Röntgendiagnostikgerät zur Erstellung von Panorama-Schichtaufnahmen, nachfolgend kurz mit PAN-Aufnahmen bezeichnet. Das Gerät enthält eine in der Höhe verstellbare Tragsäule 1, an der eine Dreheinheit 2 gehaltert ist, die Träger einerseits einer Röntgenstrahlenquelle 3 mit Primärblende 6 und diametral dazu andererseits einer Detektoranordnung 4 ist. Mit 5 ist eine Kopfhalte- und Positioniereinrichtung bezeichnet, mit der in bekannter Weise der Patientenkopf in einer definierten Position fixiert werden kann. Aufbau sowie Verstellmöglichkeiten der Dreheinheit und der Kopfhalte- und Positioniereinrichtung sind bekannt und beispielsweise in der EP-0 229 308 beschrieben.

Die Detektoranordnung 4 enthält drei in einem geringen Abstand übereinander angeordnete, röntgenstrahlenempfindliche CCD-Elemente 7 bis 9 (Fig. 2) bestimmter Breite (B) und Länge (L). Für PAN-Aufnahmen beträgt die CCD-Breite (x-Richtung) typischerweise 5 bis 10 mm, die Sensorlänge (y-Richtung) insgesamt 150 mm. Die TDI-Richtung, in der die Ladungspakete innerhalb des CCD's transportiert werden, ist durch einen Pfeil angegeben. In der Breite sind mehrere Pixelreihen nebeneinander angeordnet.

In Fig. 3 ist das für eine Reihe von Pixeln der CCD-Elemente 7 bis 9 tatsächlich vorliegende Dunkelstromprofil anhand der Dunkelstromwerte DS jedes einzelnen Pixels der CCD-Elemente 7 bis 9 dargestellt. Die Dunkelstromwerte der einzelnen Pixel untereinander können beträchtlich voneinander abweichen, wodurch die Profile stark voneinander abweichen können. Herstellungsbedingt sind besonders im Randbereich hohe Dunkelstromwerte vorhanden, da das Aussägen des Sensormaterials Veränderungen im Material bewirkt. Dadurch kommt es in der Umgebung der Übergänge zwischen zwei benachbarten CCD-Elementen 7, 8, 9 zu Sprüngen.

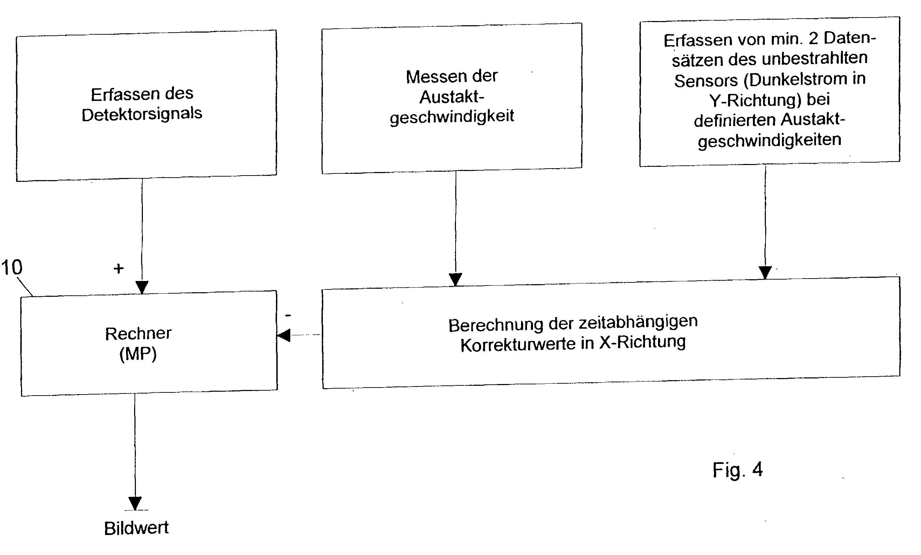

Wie aus dem Ablaufschema nach Fig. 4 hervorgeht, werden die erfaßten Bildinformationen, als Detektorsignal S, bestehend aus dem ausgetakteten Bildsignalen BS mit den Dunkelstromsignalen D5, ausgelesen und in einen Rechner 10 gegeben. Gleichzeitig werden die dazugehörigen Austaktgeschwindigkeit gemessen und festgehalten. Wie bereits angesprochen, werden vor Beginn einer Aufnahme oder nach einer Aufnahme mindestens zwei Datensätze vom unbestrahlten Sensor bei definierten Austaktgeschwindigkeiten v₁, v₂ erfaßt. Die so gewonnenen Informationen werden einer Berechnung durch Abschätzung unterzogen, wobei dabei ein für jeden Bildpunkt berechneter Dunkelstromwert DW bestimmt wird. In Verbindung mit den Austaktzeiten werden zeitabhängige Korrekturwerte ermittelt und dem Rechner 10 zur Kompensation des Dunkelstromes zur Verfügung gestellt. Der Rechner 10 liefert sodann im Dunkelstromverhalten verbesserte Bildwerte BW, welche in bekannter Weise zu einem Bild aufbereitet werden, das in bekannter Weise auf einem Monitor wiedergegeben werden kann.

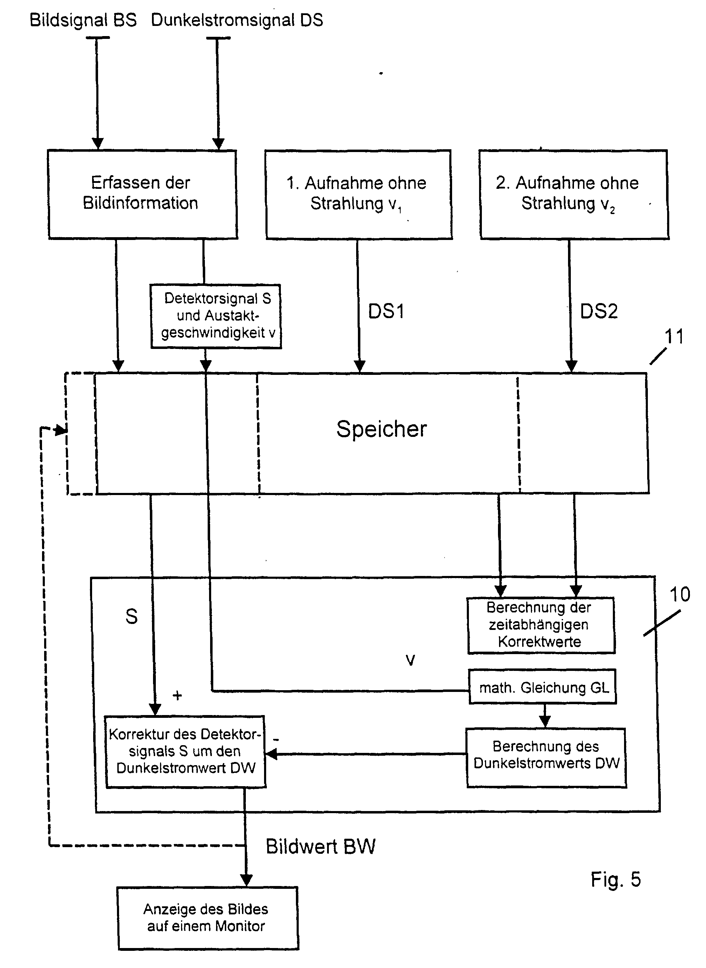

In Fig. 5 ist das Ablaufschema derart verfeinert, daß eine Speichereinheit 11 vorgesehen ist, in der die aus Bildsignal BS und Dunkelstromsignal DS bestehenden Bildinformationen als Detektorsignal S unter Erfassung der Austaktgeschwindigkeit v abgelegt sind. In dem Speicher 11 sind weiterhin das zu jedem Pixel gehörige Dunkelstromsignal DS1, DS2 der mindestens zwei Aufnahmen unterschiedlicher Austaktgeschwindigkeit v₁, v₂ abgelegt, die zur Berechnung des zeitabhängigen Dunkelstromwertes DW zur Korrektur des Detektorsignals S herangezogen werden. Durch die Austaktgeschwindigkeit v ist ein direkter Bezug zu der Integrationszeit gegeben, da bei hoher Austaktgeschwindigkeit v die Integrationszeit gering ist und umgekehrt. Die Austaktgeschwindigkeit selbst steht in einer Beziehung zu der Geschwindigkeit der Bewegung des Sensors.

In dem Rechner 10 erfolgt die Berechnung des Dunkelstromwertes DW in Abhängigkeit der Integrationszeit, wobei die Dunkelstromsignale DS1, DS2 zur Erzeugung einer Gleichung erster Ordnung herangezogen und der Dunkelstromwert DW durch Inter- oder Extrapolation ermittelt wird. Wird die Anzahl der Aufnahmen ohne Strahlung mit unterschiedlichen Austaktgeschwindigkeiten v₁, ... vₙ, erhöht, so nimmt die Zahl der Stützstellen zur Erzeugung einer mathematischen Gleichung höherer Ordnung zu.

Zur Korrektur des Detektorsignals wird nun der zur Austaktgeschwindigkeit v gehörige Dunkelstromwert DW über die mathematische Gleichung GL für jedes Pixel berechnet, wobei der Dunkelstromwert DW von dem Detektorsignal S abgezogen wird. Der derart berechnete Bildwert BW wird auf einem Monitor 12 angezeigt und gegebenenfalls in dem Speicher 11 abgespeichert. Selbstverständlich kann der Bildwert auch direkt ausgedruckt werden oder anderweitig verwendet werden.

## Patentansprüche

1. Verfahren zur Kompensation des Dunkelstroms eines mehrere Pixel mit einem individuellen Dunkelstromverhalten aufweisenden elektronischen Sensors, wobei aus einer Strahlenquelle (3) eine Strahlung zur Erzeugung eines Bildsignals (BS) auf eine den Sensor enthaltende Detektoranordnung (4) gerichtet ist und eine Aufnahme mit unterschiedlicher Austaktgeschwindigkeit (v) durch Auslesen des in den Pixeln des Sensors vorhandenen Detektorsignals (S) erstellt wird, dadurch gekennzeichnet, daß vor Beginn oder nach dem Ende der Aufnahme der Sensor ohne Strahlung mit wenigstens zwei unterschiedlichen Austaktgeschwindigkeiten (v₁, v₂) ausgelesen und damit für jede Pixel wenigstens zwei Dunkelstromsignale (DSI, DS2) erfaßt werden, daß anschließend die ausgelesenen Dunkelstromsignale (DS1, DS2) zur Berechnung eines Dunkelstromwert (DW) einzelner Pixel des Sensors in Abhängigkeit der Austaktgeschwindigkeit (v) herangezogen werden und daß anschließend mit dem jedem Pixel zugeordneten Dunkelstromwert (DW) eine Korrektur des mit dem Dunkelstromsignal (DS) überlagerten Detektorsignals (S) zur Ermittlung des Bildwertes (BW) vorgenommen wird.

2. Verfahren nach Anspruch 1, dadurch gekennzeichnet, daß der elektronische Sensor, aus dem die Meßsignale ausgelesen werden, ein CCD-Sensor ist.

3. Verfahren nach Anspruch 1 oder 2, dadurch gekennzeichnet, daß der Sensor eine zweidimensionale Pixelmatrix aufweist und im TDI-Modus betrieben wird.

4. Verfahren nach einem der Ansprüche 1 bis 3, dadurch gekennzeichnet, daß die Aufnahme mittels eines in mehrere voneinander räumlich abgesetzte Bereiche (7, 8, 9) unterteilten Sensors erfolgt.

5. Verfahren nach einem der Ansprüche 1 bis 4, dadurch gekennzeichnet, daß für die Berechnung der Abhängigkeit des Dunkelstromwerts (DW) von der Austaktgeschwindigkeit (v) anhand der mindestens zwei ausgelesenen Dunkelstromsignale (DS1, DS2) eine rechnerische Beziehung hergstellt wird und daß der einem Pixel zugeordnete Dunkelstromwert (DW) mittels einer Inter- oder Extrapolation entsprechend der rechnerischen Beziehung in Abhängigkeit der tatsächlichen Auftaktgeschwindigkeit berechnet wird.

6. Verfahren nach einem der Ansprüche 1 bis 5, dadurch gekennzeichnet, daß eine zahnärtzliche Röntgenaufnahme erstellt wird.

7. Verfahren nach einem der Ansprüche 1 bis 6, dadurch gekennzeichnet, daß eine Erstellung von zahnärztlichen Panorama- oder cephalometrischen Schichtaufnahmen mit einem Röntgendiagnostikgerät erfolgt, welches eine Dreheinheit (2) mit einer Strahlenquelle (3) und einer diametral dazu angeordenten Detektoranordnung (4) mit mindestens einem elektronischem Sensor enthält.

8. Verfahren nach einem der Ansprüche 1 bis 7, dadurch gekennzeichnet, daß die beim Auslesen des Sensors entstehenden Detektorsignale (S) in einer Speichereinheit (11) abgespeichert werden und daß in einem nachfolgenden Schritt die Korrektur des mit dem Dunkelstromsignal (DS) überlagerten Detektorsignals (S) zur Ermittlung der Bildwerts (BW) in einer mit der Speichereinheit (11) verbundenen Recheneinheit (10) vorgenommen wird.

9. Verfahren nach einem der Ansprüche 1 bis 7, dadurch gekennzeichnet, daß vor der Aufnahme wenigstens zwei Dunkelstromsignale (DS1, DS2) erfaßt werden, daß während der Aufnahme anhand der Austaktgeschwindigkeit (v) ein Dunkelstromwert (DW) berechnet wird, daß das Detektorsignal (S) um den Dunkelstromwert (DW) korrigiert wird und daß der so erhaltene Bildwert (BW) angezeigt und/oder abgespeichert wird.

## Claims

1. Method for compensating for the dark current of an electronic sensor having a plurality of pixels with an individual dark-current response, radiation for producing an image signal (BS) being directed from a beam source onto a detector arrangement (4) containing the sensor, and a recording being taken with different clock-out rate (v) by reading the detector signal (S) present in the pixels of the sensor, characterized in that, before the beginning or after the end of the recording, the sensor is read out without radiation with at least two different clock-out rates (v1, v2) and at least two dark-current signals (DC1, DC2) are thereby picked up for each pixel, in that the dark-current signals (DC1, DC2) that have been read out are then used to calculate a dark-current value (DV) of individual pixels of the sensor as a function of the clock-out rate (v), and in that a correction is then made, using the dark-current value (DV) associated with each pixel, to the detector signal (S) with the dark-current signal (DC) superimposed on it in order to calculate the picture value (BW).

2. Method according to Claim 1, characterized in that the electronic sensor from which the measurement signals are read out is a CCD sensor.

3. Method according to Claim 1 or 2, characterized in that the sensor has a two-dimensional pixel matrix and is driven in TDI mode.

4. Method according to Claims 1 to 3, characterized in that the recording is made using a sensor divided into a plurality of regions (7, 8, 9) spatially separated from one another.

5. Method according to Claims 1 to 4, characterized in that for calculating the dependency of the dark-current value (DV) on the clock-out rate (v) with the aid of the at least two dark-current signals (DC1, DC2) that are read out, a computed relationship is produced, and in that the dark-current value (DV) assigned to a pixel is calculated by means of an inter- or extrapolation corresponding to the computed relationship as a function of the actual clock-out rate.

6. Method according to Claims 1 to 5, characterized in that a dental X-ray recording is taken.

7. Method according to Claims 1 to 6, characterized in that dental panorama or cephalometric tomographs are taken using an X-ray diagnosis instrument which contains a rotation unit (2) having a beam source (3) and, arranged diametrically with respect to it, a detector arrangement (4) having at least one electronic sensor.

8. Method according to Claims 1 to 7, characterized in that the detector signals (S) obtained when reading out the Sensor are stored in a memory unit (11), and in that the correction to the detector signal (S) with the dark-current signal (DC) superimposed on it to ascertain the picture value (PV) is carried out in a subsequent step in a computer unit (11) connected to the memory unit.

9. Method according to Claims 1 to 7, characterized in that at least two dark-current signals (DC1, DC2) are picked up before the recording, in that a dark-current value (DV) is calculated during the recording with the aid of the clock-out rate (v), in that the detector signal (S) is corrected by the dark-current value (DV) and in that the picture value (PV) thus obtained is displayed and/or stored.

## Revendications

1. Procédé de compensation du courant d'obscurité d'un capteur électronique comportant plusieurs pixels ayant un comportement propre de courant d'obscurité, un rayonnement destiné à la production d'un signal (BS) d'image étant dirigé d'une source (3) de rayonnement sur un dispositif (4) à détecteur comportant le capteur et une prise de vue étant prise à des vitesses (v) de cadence différentes en lisant le signal (S) du détecteur présent dans les pixels du capteur, caractérisé en ce qu'avant le début ou après la fin de la prise de vue on lit le capteur sans rayonnement à au moins deux vitesses (v₁, v₂) de cadence différentes et on relève ainsi pour chaque pixel au moins deux signaux (DS1, DS2) de courant d'obscurité, en ce qu'ensuite, on tire parti des signaux (DS1, DS2) de courant d'obscurité qui sont lus pour calculer une valeur (DW) du courant d'obscurité des pixels individuels du capteur en fonction de la vitesse (v) de cadence et en ce qu'ensuite on effectue avec la valeur (DW) de courant d'obscurité associée à chaque pixel une correction du signal (S) du détecteur superposé au signal (DS) de courant d'obscurité pour déterminer la valeur (BW) d'image.

2. Procédé suivant la revendication 1, caractérisé en ce que le capteur électronique dans lequel on lit les signaux de mesure est un capteur CCD.

3. Procédé suivant la revendication 1 ou 2, caractérisé en ce que le capteur comporte une matrice de pixel bidimensionnelle et fonctionne en mode TDI.

4. Procédé suivant l'une des revendications 1 à 3, caractérisé en ce que l'on effectue la prise de vue au moyen d'un capteur subdivisé en plusieurs domaines (7, 8, 9) décalés dans l'espace les uns par rapport aux autres.

5. Procédé suivant l'une des revendications 1 à 4, caractérisé en ce que, pour le calcul de la variation de la valeur (DW) du courant d'obscurité en fonction de la vitesse (v) de cadence, on élabore au moyen des au moins deux signaux (DS1, DS2) de courant d'obscurité qui sont lus une relation par le calcul et en ce que l'on calcule la valeur (DW) de courant d'obscurité associée à un pixel au moyen d'une interpolation ou d'une extrapolation correspondant à la relation obtenue par le calcul en fonction de la vitesse de cadence effective.

6. Procédé suivant l'une des revendications 1 à 5, caractérisé en ce qu'on élabore une prise de vue aux rayons X de médecine dentaire.

7. Procédé suivant l'une des revendications 1 à 6, caractérisé en ce que l'on effectue une élaboration de tomographie panoramique ou céphalométrique pour la médecine dentaire à l'aide d'un appareil de diagnostic aux rayons X qui comporte une unité (2) tournante ayant une source (3) de rayonnement et un dispositif (4) à détecteur disposé de manière diamétralement opposée et ayant au moins un capteur électronique.

8. Procédé suivant l'une des revendications 1 à 7, caractérisé en ce que les signaux (S) du détecteur formés lors de la lecture du capteur sont mémorisés dans une unité (11) de mémoire et en ce que dans un stade venant ensuite, on effectue la correction du signal (S) de détecteur superposé au signal (DS) de courant d'obscurité, pour la détermination de la valeur (BW) d'image dans une unité (10) de calcul reliée à l'unité (11) de mémoire.

9. Procédé suivant l'une des revendications 1 à 7, caractérisé en ce qu'avant la prise de vue, on relève au moins deux signaux (DS1, DS2) de courant d'obscurité, en ce que pendant la prise de vue on calcule au moyen de la vitesse (v) de cadence une valeur (DW) de courant d'obscurité, en ce que l'on corrige le signal (S) du détecteur de la valeur (DW) de courant d'obscurité et en ce que l'on affiche et/ou mémorise la valeur (DW) d'image ainsi obtenue.
